# EUROPEAN PATENT APPLICATION

(11) **EP 4 659 672 A1**
(43) Date of publication of application: **10.12.2025**
(21) Application number: 24180869.0
(22) Date of filing: 07.06.2024
(51) Int. Cl.: A61B 5/28, A61B 5/282, A61B 5/00

(54) **TEXTILE ELECTRODE**

(71) Applicant: Universiteit Gent, 9000 Gent (BE); Imec VZW, 3001 Leuven (BE)
(72) Inventor: Veske-Lepp, Paula, 9052 Gent (BE)
(74) Representative: IP HILLS NV

(57) **Abstract**

A textile electrode (1, 2, 3) having an inner side (110, 210, 310) for contacting a skin surface (111, 211, 311) and an outer side (120, 220, 320) for bordering with an environment, the textile electrode comprising a thermoplastic layer (130, 230, 330) comprising a plurality of closed, air-filled cavities (135, 235, 335) delineating protruding portions (136, 236, 336) on the inner side of the electrode for contacting the skin surface; and a textile layer (140, 240, 340) laminated to the thermoplastic layer towards the outer side of the textile electrode.

## Description

### Technical field

The present invention generally relates to a textile electrode and a method for manufacturing a textile electrode.

### Background

Smart textiles are fabrics with integrated electronics. Smart textile systems allow sensing of signals on a body of a human or of an animal via clothing.

Some challenges of smart textiles include the following. Smart textile systems require providing a stable conductive path to the point of sensing. This may include a need to keep the electronics in place during measurements. The fabric may also be subject to wear and tear, e.g. due to washing. The integrated electronics need to be able to retain its functioning under such circumstances.

In one application, an electrocardiographic signal is measured by means of a textile electrode making contact with the skin, e.g. near the heart.

There are specific requirements for textile electrodes to function properly in smart textiles. First, providing a good contact with the skin is crucial for obtaining a reliable electrical signal. Soft electrodes can be used due to their ability to adapt to the shape of the skin and due to the comfort it provides to a wearer of the electrode. A drawback of soft electrodes is that they may partially loose contact with the skin more easily compared to hard electrodes, in particular when the wearer of the electrode is in motion. Second, the textile electrode preferably stays in place for a stable measurement. Furthermore, the textile electrode must resist wear and tear and be repeatedly washable. Thus, the textile electrode needs to retain its functionality over frequent rubbing, abrasion and/or washing cycles and the like. In addition, manufacturing of the textile electrode should be efficient and scalable.

### Summary

The present invention aims to alleviate at least some of the above mentioned obstacles.

The scope of protection sought for various embodiments of the invention is set out by the independent claims.

The embodiments and features described in this specification that do not fall within the scope of the independent claims, if any, are to be interpreted as examples useful for understanding various embodiments of the invention.

According to a first aspect, a textile electrode is provided. The textile electrode has an inner side for contacting a skin surface and an outer side for bordering with an environment. The textile electrode comprises a thermoplastic layer and a textile layer. The thermoplastic layer comprises a plurality of closed, air-filled cavities delineating protruding portions on the inner side of the electrode for contacting the skin surface. The textile layer is laminated to the thermoplastic layer towards the outer side of the textile electrode.

The thermoplastic layer is made up of solid, thermoplastic material that traps air in the cavities. The protruding portions protrude outwardly from the cavities towards the inner side of the electrode. The protruding portions follow the shape of the cavities on the inner side of the electrode. As such, the shape of the protruding portions corresponds to the shape of the cavities.

The air-filled cavities of the thermoplastic layer provide the electrode with a shape-retaining property. After pressing the electrode, the electrode may temporarily deform as the air inside the cavities is compressed. After a pressing force is released, the electrode retakes its original shape due to the trapped air, which then re-expands. In other words, the electrode remains flexible yet keeps its strength while it is strained as a result of movements of the wearer during use. As such, the thermoplastic layer has a firmness that enables the electrode to provide a good contact with the skin.

Further, the contact surface is provided by the tips of the protruding portions. As such, the contact surface is made up of a collection of separate, discontinuous contact regions. In other words, by cause of the protruding portions, not the entire inner area of the electrode makes contact with the skin.

This results in an adaptable contact surface between the electrode and the skin. The flexibility results from the ability of the tips of the protruding portions to compensate for local stretching or accumulation of the skin during movement. As such, the textile electrode maintains a good contact with the skin. Even in motion, the electrode intrinsically tends towards retaining contact with the skin with all the tips of the protruding portions.

Furthermore, the flexibility provided by the tips allows absorbing tension caused by movement of the skin surface. As a result, the textile electrode has a tendency to stay in one place on the skin.

In addition, due to the disjoint points of contact, the electrode may be perceived as less sticky on the skin and may be more comfortable to wear.

The thermoplastic layer omits a necessity of any foam or other filler material because of the presence of the cavities. As such, less material is needed for the electrode to be firm. Also, the thermoplastic layer can be made up of a single material type. This results in an efficient manufacturing process.

Further, the textile layer provides the textile electrode a resistance against repeated washing and other external wear. The textile layer may be non-conductive, thereby providing an isolation of the conductive portions of the textile electrode. The textile layer may also be conductive.

According to further example embodiments, the thermoplastic layer comprises an outer thermoplastic layer and an inner thermoplastic layer having unmatching surfaces such that the closed, air-filled cavities occur between the outer and inner thermoplastic layers.

The surfaces are unmatching in the sense that they have different surface shapes, thereby leaving gaps between them when brought together, e.g. after the lamination.

By providing separate inner and outer thermoplastic layers, the cavities can be formed in a controlled and uncomplicated manner during manufacturing.

According to further example embodiments, the inner thermoplastic layer is thermoformed to rigidly provide the protruding portions.

Thermoforming is the process of heating up a planar layer or sheet of material, followed by pressing a mould thereon to transform the planar layer into a shaped layer of the material.

By thermoforming, surfaces for the cavities and corresponding protruding portions are efficiently formed on the inner thermoplastic layer.

The thermoformed inner thermoplastic layer provides firm protruding portions for a good contact with the skin. In addition, the thermoformed inner thermoplastic layer retains its shape when the textile layer is laminated to the thermoplastic layer.

According to further example embodiments, the outer thermoplastic layer has a planar surface to provide a planar textile layer for the electrode.

The textile layer laminated onto the thermoplastic layer takes the shape of the outer thermoplastic layer. Thus, by providing a flat outer thermoplastic layer, the textile layer laminated on the outside of the textile electrode is also planar.

By providing a flat textile layer, the textile electrode can be easily integrated within a smart textile system. A piece of clothing can unobtrusively comprise the textile electrode. This may add to the comfort of the wearer.

According to further example embodiments, the outer thermoplastic layer is thermoformed to provide an uneven textile layer for the electrode.

By providing a textured outer thermoplastic layer, the textile layer laminated on the outside of the textile electrode is also uneven. By providing an uneven textile layer, the textile layer can protrude outwardly from the textile electrode.

This can be useful to easily spot or feel where the textile electrode is located within a piece of clothing. Additionally, such an uneven textile layer could be used for keeping the textile electrode in place. For example, a corresponding curvature could be provided on the inside of fabric of the smart textile system to receive protruding ends of the textile layer.

According to further example embodiments, the textile electrode further comprises a protective layer on an outer side of the textile layer.

The protective layer may cover the entire textile layer. Alternatively, the protective layer may only cover part of the textile layer. For example, the protective layer may occur at a region near the edge circumventing the textile electrode. As another example, the protective layer may occur at the corners of the textile electrode.

The protective layer strengthens the textile electrode, while retaining the advantage of washability provided by the textile layer. As such, the protective layer further adds to the resistance of the textile electrode against wear and tear. In case the protective layer deteriorates over time due to repeated washing and wearing, the textile layer still provides the necessary protection of the textile electrode.

According to further example embodiments, the textile electrode further comprises a conductive layer on an inner side of the thermoplastic layer.

The conductive layer is configured to make contact with the skin for sensing of a signal on the skin surface. The conductive layer may, for example, comprise a conductive spray or a knitted layer of conductive yearn.

According to further example embodiments, the textile electrode further comprises a positioning layer around the protruding portions on an inner side of the thermoplastic layer for holding the textile electrode in place during use.

The positioning layer comprises a material that provides adhesion with the skin, thereby keeping the textile electrode at a fixed position. The positioning layer is positioned on the inner side of the textile electrode, in a region where the protruding portions do not occur. As such, the positioning layer does not interfere with the sensing function of the textile electrode.

According to a second aspect, there is provided a method for manufacturing a textile electrode according to the first aspect. The textile electrode has an inner side for contacting a skin surface and an outer side for bordering with an environment. The method comprises:
- providing a first layer having a textile sublayer and an outer thermoplastic sublayer laminated together;
- providing a second layer, comprising at least an inner thermoplastic sublayer;
- thermoforming the second layer, such that an uneven surface is formed; and
- laminating the first layer and the second layer together with the outer and inner thermoplastic sublayers facing each other, thereby obtaining closed, air-filled cavities between the outer and inner thermoplastic sublayers.

The outer thermoplastic sublayer provides the textile sublayer with support. By providing the outer thermoplastic sublayer laminated onto the textile sublayer, the textile can be wielded more easily during the following manufacturing steps. The outer thermoplastic sublayer also already provides the outer part of the thermoplastic layer.

The method allows for an efficient production process and a frugal use of materials. In addition, only a laminating device and a thermoforming device are required to obtain the textile electrode.

According to further example embodiments, the providing of the first layer further comprises laminating the outer thermoplastic sublayer and the textile sublayer together.

The lamination to obtain the first layer can be done during the manufacturing process. Alternatively, pre-laminated textile can be used.

According to further example embodiments, the method further comprises providing a conductive layer on the inner side of the electrode.

The conductive layer is provided to make contact with the skin for sensing of a signal on the skin surface. The providing of the conductive layer may, for example, comprise spraying a conductive layer on the inner side of the electrode. As another example, the providing of the conductive layer may comprise providing a textile layer of conductive yearn on the inner side of the electrode.

According to further example embodiments, the second layer further comprises the conductive layer laminated onto the inner thermoplastic sublayer.

According to further example embodiments, the method further comprises providing the conductive layer onto the thermoformed second layer.

According to further example embodiments, the method further comprises laminating a positioning layer around the protruding portions onto the inner side of the textile electrode for holding the textile electrode in place during use.

According to further example embodiments, the method further comprises providing a snap fastener through the layers of the textile electrode to provide an electrical contact from the outer side to the conductive layer.

A snap fastener has two fixating elements that can interlock with each other. The two elements are pushed onto each other while piercing the layers of the textile electrode between them, preferably in a region without protruding portions. Thereby, a conductive path is created from the conductive layer to the snap fastener ends. As such, the snap fastener element at the outer side of the electrode provides a convenient connection point for obtaining a signal sensed at the skin surface.

According to further example embodiments, the method further comprises:
- providing a protective layer; and
- laminating the protective layer on the textile layer.

### Brief Description of the Drawings

Fig. 1 illustrates a textile electrode according to example embodiments;
Fig. 2 illustrates a textile electrode having a thermoformed outer thermoplastic layer according to example embodiments;
Fig. 3 illustrates a textile electrode having a protective layer and a positioning layer according to example embodiments;
Fig. 4 illustrates textile electrodes having different configurations according to example embodiments; and
Fig. 5 illustrates a method for manufacturing a textile electrode according to example embodiments.

### Detailed Description of Embodiment(s)

The present disclosure relates to a textile electrode for sensing or capturing signals on the human or animal body. For example, an electrode can be used to obtain an electrocardiographic signal via the skin surface.

An electrode comprises a conductive portion. On one end, the conductive portion can make contact with the body to pick up an electrical signal. On the other end, the conductive portion can provide the sensed signals to a circuitry provided in an external device, e.g. a processing unit, for read-out and further processing. Thereto, the conductive portion can include a contact terminal for galvanically connecting to the electrode. As such, the conductive portion provides a conductive path to capture a signal and to lead it outwardly from the electrode. Further, an electrode generally also comprises a non-conductive portion for providing electrical isolation and a firmness to obtain stable measurements.

A textile electrode is an electrode that can operate as a wearable embedded within a textile. For example, a textile electrode can be integrated into a shirt for wearing by a person during exercising. As another example, a textile electrode can be integrated into a rug to be worn by an animal. A textile electrode is usually made of a soft material that adapts to the shape of a textile fabric into which it is embedded, and to movements of a wearer and the skin surface.

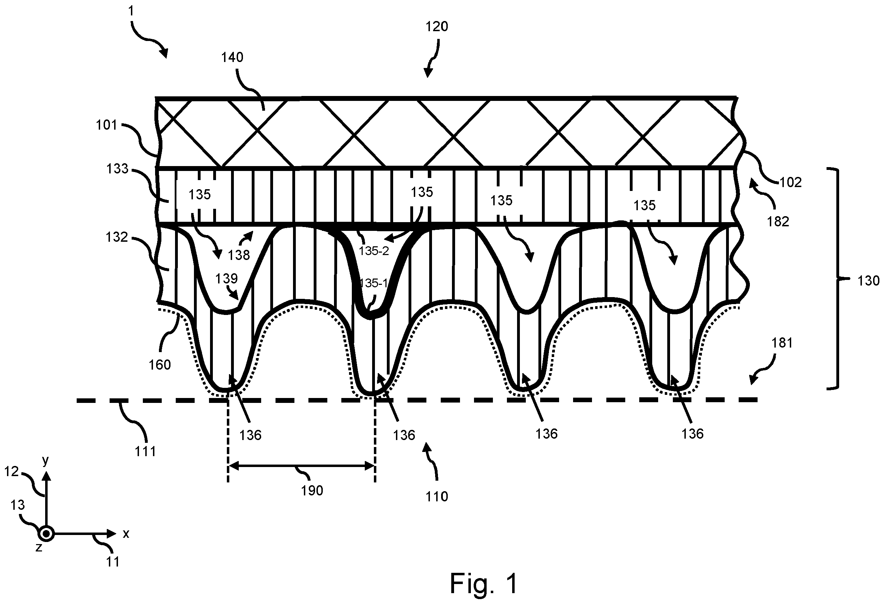

Figure 1 shows a cross-section of a textile electrode 1 according to example embodiments. The textile electrode 1 has an inner side 110 for contacting skin surface 111, e.g. of a person or an animal. The skin surface 111 may, for example, pertain to a skin region near the heart where the electrode can then sense electrical signals produced by the heart. The textile electrode 1 has an outer side 120 for bordering with an environment. The outer side 120 may, for example, be partially exposed to the air, and/or, may be attached to textile clothing.

An x-direction 11 defines a length of the textile electrode 1. A y-direction 12 defines a height of the textile electrode 1, wherein the y-axis points towards the outer side 120. A z-direction defines a width of the textile electrode 1. Figure 1 shows a cross-section of the textile electrode 1 in an xy-plane defined by horizontal x-direction 11 and vertical y-direction 12 to show the stack of layers. The textile electrode 1 has a surface covering the skin that is defined along the xz-plane. The textile electrode 1 comprises stacked layers 140, 132, 133, and 160 of material. Layers 132, 133 further form a combined layer 130.

In correspondence to the inner side 110 and outer side 120 of the textile electrode 1, each such layer may be referred to as having a corresponding inner side, i.e. facing the skin surface, and a corresponding outer side, i.e. facing the environment. For example, layer 130 has an inner side 181 and an outer side 182. The textile electrode 1 may continue further to the left side and to the right side. This is indicated by undulating borders 101 and 102 respectively. The textile electrode 1 may comprise additional and/or different layers around the edges.

The textile electrode 1 comprises a thermoplastic layer 130 for contacting the skin surface 111. The thermoplastic layer 130 comprises a plurality of closed, air-filled cavities 135. Such an air-filled cavity 135 is formed by an enclosed surface 135-1, 135-2. The enclosed surface is formed by first surface 135-1 towards the inner side 110 and second surface 135-2 towards the outer side 120. The cavity 135 delineates a protruding portion 136 that follows the shape of the first surface 135-1, thereby protruding towards the inner side 110. The air-filled cavities 135 provide the electrode with firmness as well as shape-retention, both contributing to a stable measurement. Tips of the protruding portions 136 make contact with the skin surface 111. The tips of the protruding portions 136 are provided with an electrically conductive surface 160 for making a galvanic contact with the skin surface. The conductive surface 160 may, for example, be provided by application of a conductive spray on the inner side 110 of the textile electrode 1. Alternatively, the conductive surface 160 may be a sheet or layer of a conductive material, e.g. conductive fabric, on the inner side 110 of the textile electrode 1. To increase the electrical conductivity, the inner side 110 of the textile electrode 1 may be wetted when applying to the skin.

The thermoplastic layer 130 may further comprise adjoining inner thermoplastic layer 132 and outer thermoplastic layer 133. Cavities 135 are formed between these layers 132 and 133. The cavities 135 may result from unmatching surfaces 138, 139 of the respective adjoining inner and outer thermoplastic layers 132, 133. The surface 138 of the outer thermoplastic layer 133 may, for example, be a flat surface. Such a flat surface may, for example, be formed through lamination of thermoplastic material. Inner thermoplastic layer 132 may, for example, have a wavy surface. Such a curved or wavy surface may, for example, be formed by thermoforming a sheet of thermoplastic material. By thermoforming, rigid protruding portions 136 are provided. In other words, the protruding portions hold their shape upon cooling down after the thermoforming. Alternatively, the thermoplastic material may be otherwise manipulated under heated conditions, or may be carved.

Other textured surfaces may also be formed, on either or both of the inner and outer thermoplastic layers 132, 133 in order to obtain the closed, air-filled cavities 135. The protruding portions may, for example, have a rectangular-shaped cross-section, such that the tips have a flat, horizontal surface. This may provide a larger contact area with the skin 111 for a more stable measurement.

Further, thermoplastic layers 132, 133 may have a repetitive surface pattern. For example, the protruding portions 136 may be repeated at regular intervals. For example, the protruding portions may be repeated in the x-direction according to a fixed inter-portion distance 190. Optionally, the protruding portions may be repeated in z-direction 13 according to a second inter-portion distance. The second inter-portion distance may be the same as distance 190.

Alternatively, the surface pattern of either of the inner and outer thermoplastic layers 132, 133 may be irregular, i.e. spatially non-periodic. For example, the thermoplastic layers 132, 133 may have protruding portions 136 that are irregularly spaced. This may allow for tailored arrangements, e.g. when it may be useful to emphasise one or more regions of interest within the area of the textile electrode 1. A multitude of protruding portions may, for example, be clustered more closely together in such a region of interest.

The thermoplastic layer 130 may comprise any thermoplastic material or a combination of thermoplastic materials. The thermoplastic layer 130 may, for example, comprise thermoplastic polyurethane.

The textile electrode 1 further comprises a textile layer 140 on the outer side 120 of the electrode. The textile layer 140 is laminated to the outer side 120 of the thermoplastic layer 130. The textile layer 140 is planar since it is provided onto the outer thermoplastic layer 133 which also has a planar surface 138. The textile layer 140 provides protection for the other inner layers of the electrode 1. In particular, the textile layer 140 shields the thermoplastic layer 130 from wear and tear, e.g. caused by washing. The textile electrode may be washed either separately or when incorporated into clothing. Additionally, by the textile layer 140, the conductive surface 160 of the protruding portions 136 is shielded during contact with the skin surface 111 when in use. This further contributes to a stable measurement.

The textile layer 140 may comprise any textile material or any combination of textile materials. The textile layer 140 may, for example, comprise one or more layers of knitted fabric. The textile layer 140 is preferably non-conductive to provide adequate shielding, yet may also comprise a conductive material.

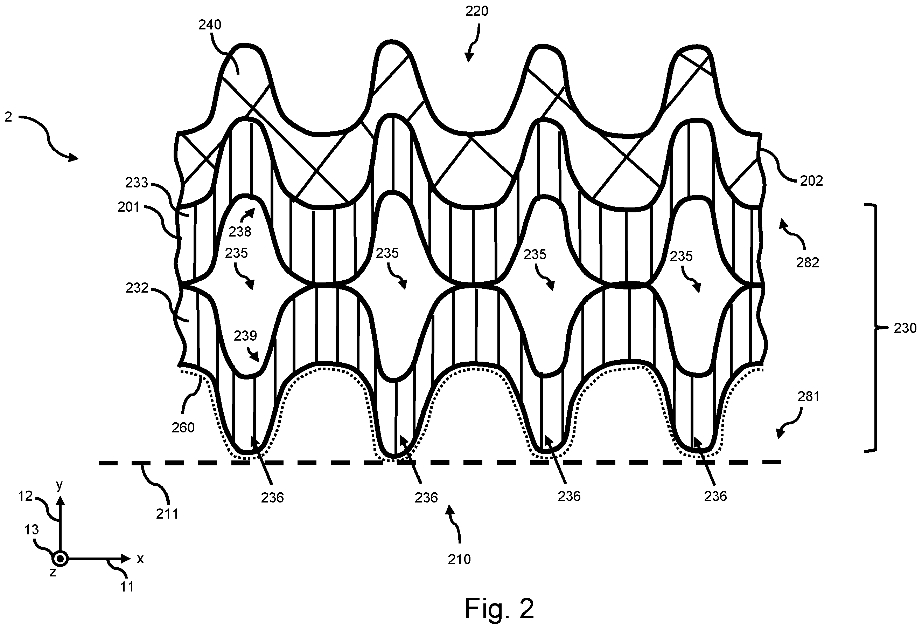

Figure 2 shows a cross-section of a textile electrode 2 according to example embodiments. Similar to Figure 1, the textile electrode 2 has an outer side 220 and an inner side 210 for contacting skin surface 211. Further, the textile electrode 2 comprises a textile layer 240 and a thermoplastic layer 230 having an outer thermoplastic layer 233 and an inner thermoplastic layer 232. The thermoplastic layers 230 also comprises closed, air-filled cavities 235, delineating protruding portions 236 that are provided with an electrically conductive surface 260. The cavities 235 result from unmatching surfaces 238, 239 of the adjoining inner and outer thermoplastic layers 232, 233. The textile electrode 2 may continue further to the left side and to the right side as indicated by undulating borders 201 and 202 respectively.

The outer thermoplastic layer 233 has a curved shape, e.g. achieved by thermoforming. The textile layer 240 is laminated to the outer thermoplastic layer 233 towards the outer side 220 of the textile electrode 2 and thereby takes the curved shape thereof. As such, the outer thermoplastic layer 233 provides the uneven textile layer 240. As a result, a textured surface is created on the outer side 220 of the textile electrode 2. This may be useful for engaging the textile electrode 2 with clothing to keep the textile electrode 2 in place. In addition, a position of the textile electrode 2 may be easily recognised, e.g. by visual inspection or by touch, even when it is placed underneath fabric.

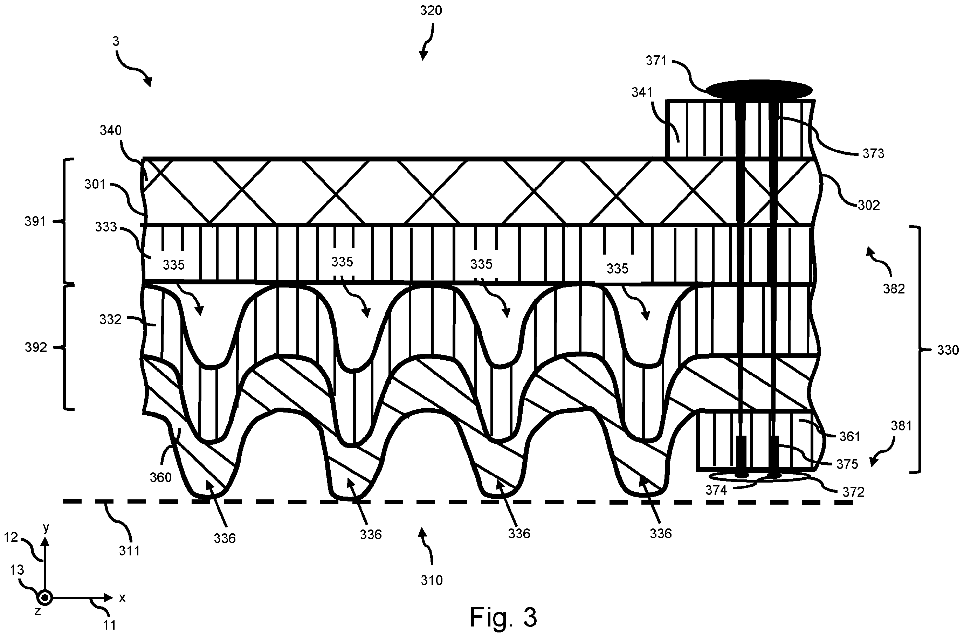

Figure 3 shows a textile electrode 3 according to example embodiments. Similar to Figure 1, the textile electrode 3 has a thermoplastic layer 330 and a textile layer 340, respectively on an inner side 310 and an outer side 320. The thermoplastic layer 330 has an inner side 381 and an outer side 382. The thermoplastic layer 330 may comprise inner thermoplastic layer 332 and outer thermoplastic layer 333, enclosing cavities 335 in between and thereby delineating protruding portions 336. The textile electrode 3 may continue further to the left side and to the right side as indicated by undulating borders 301 and 302 respectively.

The textile electrode 3 may further comprise a conductive layer 360 on the inner side 381 of the thermoplastic layer 330 for contacting skin surface 311. Thereby, the conductive layer 360 matches a shape of a surface at the inner side of the inner thermoplastic layer 332. As such, the tips of the protruding portions 336 comprise the conductive layer 360 in addition to the inner thermoplastic layer 332. The conductive layer 360 may, for example, comprise knitted conductive yearns and may, for example, be thermoformed together with the inner thermoplastic layer 332 to obtain a layer 392.

The textile layer 340 is provided onto the outer thermoplastic layer 333 and takes the flat shape thereof. The textile layer 340 and the outer thermoplastic layer 333 may, for example, be laminated together, thereby forming a layer 391.

The textile electrode 3 is further provided with a snap fastener 371, 372 to provide an electrical read-out terminal 371. The snap fastener comprises a first and second part 371, 372 that interlock when pressed onto each other. The first part 371 is provided on the outer side 320 for read-out and the second part 372 is provided on the inner side 310 of the textile electrode 3. Upon pressing the parts 371, 372 of the snap fastener together, the layers of the textile electrode 3 are pushed together. Also, the conductive portion 360 of the thermoplastic layer 330 makes contact with the parts 371, 372 upon pressing the snap fastener together. To this end, the part 371 may, for example, comprise protruding points 373, and the part 372 may, for example, comprise complementary holes 374 for holding the points. The snap fastener 371, 372 may comprise one or more hooking elements 375 for a strong interlocking. As such, a reliable conductive path is provided from the skin surface 311, through the conductive layer 360 to the fastener 371, 372.

The textile electrode 3 may further comprise a protective layer 341 on an outer side of the textile layer 340, e.g. on top of the textile layer 340. The protective layer 341 may, for example, be another thermoplastic layer. The protective layer 341 may, for example, comprise thermoplastic polyurethane. The protective layer 341 further shields the inner side of the textile electrode 3 in addition to the shielding provided by textile layer 340. The protective layer 341 may be provided along an edge or at the corners of the textile electrode 3, e.g. borders in the xz-plane. The protective layer 341 may be provided in a material that is easy for the snap fastener 371, 372 to engage with, e.g. a thermoplastic material or a textile material. The protective layer 341 may be provided in a region where a snap fastener 371, 372 is provided. As a result, the fastening may be performed more quickly and a more cohesive connection may be obtained. In Figure 3, the protective layer 341 is illustrated as occurring only in a region without the cavities 335 and protruding portions 336. Optionally, the protective layer 341 may also occur in a region with the cavities 335 and the protruding portions 336. The protective layer 341 may, for example, be provided along the entire textile layer 340.

The textile electrode 3 may further comprise a positioning layer 361 on the inner side 381 of the thermoplastic layer 330. The positioning layer 361 may, for example, be a thermoplastic layer. The positioning layer 361 is provided in regions around the protruding portions 336, i.e. avoiding the protruding portions. For example, the positioning layer 361 may be provided along a border or corner of the textile electrode 3, e.g. seen along the xz-plane. The positioning layer 361 aids in holding the textile electrode 3 in place during measurements when contacting the skin surface 311. The positioning layer 361 may be retracted from the skin surface 311 in a neutral state of the textile electrode 3, as shown in Figure 3. When slightly pressed, the textile electrode 3 will deform and the positioning layer 361 will contact the skin surface 311. Due to friction, the positioning layer 361 will provide resistance to movements of the textile electrode 3 along the skin surface 311. Alternatively, the positioning layer 361 may be provided so as to contact to the skin surface 311 by default, i.e. even in the neutral state when the textile electrode 3 is not being pressed.

Optionally, the protective layer 341 and the positioning layer 361 may be provided in the same regions of the textile electrode 3. Then, the protective layer 341 and the positioning layer 361 may be provided coincidentally at the outer and inner sides 320, 310 respectively. This may simplify a manufacturing process of the textile electrode 3.

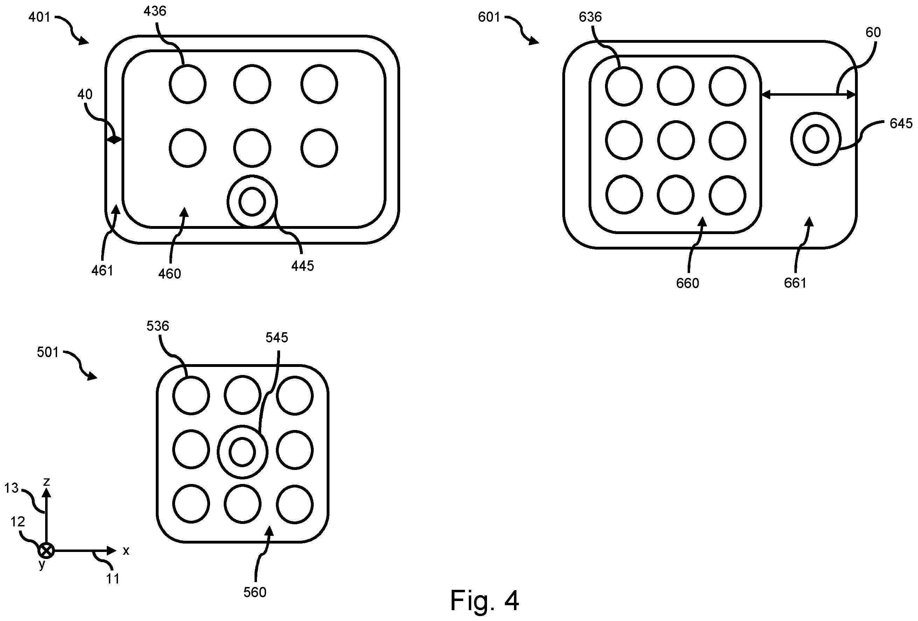

Figure 4 shows bottom views 401, 501, 601 of textile electrodes according to example embodiments. These arrangements may, for example, be provided for textile electrodes 1, 2, and 3. The textile electrodes 1, 2, 3 may have a rectangular or square surface shape and may be relatively thin. The electrodes comprises a stack of thin layers of material. For example, the textile electrodes may have a length of about 2-10 centimetres, cm, a width of about 10-15 cm and may be a few millimetres, mm, thick including air-filled cavities, e.g. up to 10-20 mm. The bottom views 401, 501, 601 show the electrodes in an xz-plane 11, 13. The bottom views 401, 501, 601 show the respective conductive layers 460, 560, 660, protruding portions 436, 536 and 636, snap fasteners 445, 545, 645, and positioning layers 461, 661.

In bottom view 401, the snap fastener 445 is directly attached onto the conductive layer 460 of the thermoplastic layer. The positioning layer 461 is provided along the entire border of the textile electrode. The width 40 of the positioning layer 461 may be constant along the border.

In bottom view 501, a square textile electrode is shown comprising the snap fastener 545 attached onto the conductive layer 560 of the thermoplastic layer. The snap fastener 545 is provided at a centre of the bottom surface of the electrode, between the protruding portions 536.

In bottom view 601, a rectangular textile electrode is shown comprising the snap fastener 645 attached onto the positioning layer 661. The positioning layer 661 is provided along the entire border of the textile electrode. A width 60 of the positioning layer 661 is larger along an edge, e.g. the right edge, where the snap fastener 645 is provided.

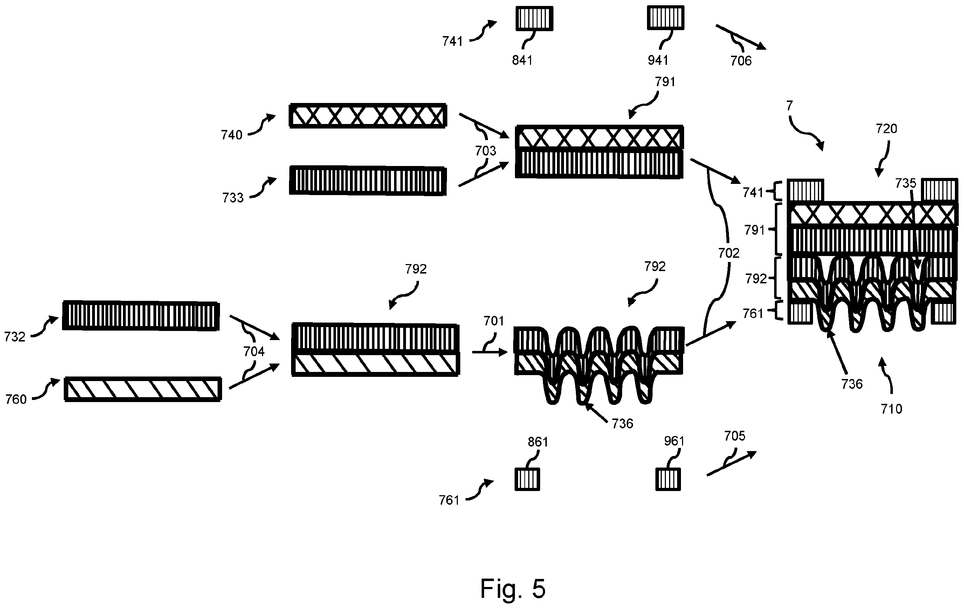

Figure 5 shows steps of a method for manufacturing a textile electrode 7 according to example embodiments. The textile electrode 7 has an inner side 710 for contacting a skin surface and an outer side 720 for bordering with an environment.

The method for manufacturing comprises providing a textile sublayer 740 and an outer thermoplastic sublayer 733. The textile sublayer 740 and the outer thermoplastic sublayer 733 are laminated 703 together to form a first layer 791. For example, a pre-laminated first layer 791 may be provided, or, the laminating 703 may be performed on separate sublayers 733, 740 during manufacturing.

The method further comprises providing a second layer 792. The second layer 792 may be formed by providing an inner thermoplastic sublayer 732 and a conductive layer 760 laminated 704 onto the inner thermoplastic sublayer 732. For example, a pre-laminated second layer 792 may be provided, or, the laminating 704 may be performed on separate sublayers 732, 760 during manufacturing. Alternatively, a conductive spray may be provided onto the inner thermoplastic layer 732 to obtain the second layer 792.

The method further comprises thermoforming 701 the second layer, such that an uneven surface is formed. By the thermoforming, protruding portions 736 are created in second layer 792.

Further, the method comprises laminating 702 the first layer 791 and the second layer 792 together while the outer and inner thermoplastic sublayers 732, 733 face each other. Thereby, closed, air-filled cavities 735 are obtained between the outer and inner thermoplastic sublayers 732, 733 and consequently, between the first and second layers 791, 792.

The method may further comprise laminating 705 a positioning layer 761 around the protruding portions 736 onto the inner side 710 for holding the textile electrode 7 in place during use. The positioning layer 761 may comprise two separate portions 861, 961 so as to provide the positioning layer 761 at two opposite edges of the textile electrode 7. Alternatively, a ring-shaped positioning layer 761 may be provided along the entire border of the textile electrode 7 similar to bottom views 401, 601 of Figure 4.

The method may further comprise laminating 706 a protective layer 741 on an outer side of the textile layer 740. The protective layer 741 may comprise two separate portions 841, 941 to provide the protective layer 741 at two opposite edges of the textile electrode 7. Alternatively, a ring-shaped protective layer 741 may be provided along the entire border of the textile electrode 7. Regions where the protective layer 741 is applied may be the same as for the positioning layer 761.

Upon obtaining the textile electrode 7, a snap fastener (not shown) may further be provided through the layers 741, 740, 733, 732, 760, 761 of the textile electrode 7. As such, an electrical contact is provided from the outer side 720 to the conductive layer 760, in particular to the conductive protruding portions 761. Optionally, the snap fastener may only be applied onto layers 740, 733, 732, 760, thereby omitting the protective and positioning layers 741, 761.

The manufacturing method shown in Figure 5 may be applied for manufacturing the textile electrodes 1, 2 and 3. To obtain textile electrode 2, an additional thermoforming step (not shown in Figure 5) is performed on first layer 791, similar to step 701.

As used in this application, the term "circuitry" may refer to one or more or all of the following: (a) hardware-only circuit implementations (such as implementations in only analog and/or digital circuitry) and (b) combinations of hardware circuits and software, such as (as applicable): (i) a combination of analog and/or digital hardware circuit(s) with software/firmware and (ii) any portions of hardware processor(s) with software (including digital signal processor(s)), software, and memory(ies) that work together to cause an apparatus, such as a mobile phone or server, to perform various functions) and (c) hardware circuit(s) and or processor(s), such as a microprocessor(s) or a portion of a microprocessor(s), that requires software (e.g., firmware) for operation, but the software may not be present when it is not needed for operation. This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A textile electrode (1, 2, 3) having an inner side (110, 210, 310) for contacting a skin surface (111, 211, 311) and an outer side (120, 220, 320) for bordering with an environment, the textile electrode comprising:
- a thermoplastic layer (130, 230, 330) comprising a plurality of closed, air-filled cavities (135, 235, 335) delineating protruding portions (136, 236, 336) on the inner side of the electrode for contacting the skin surface; and
- a textile layer (140, 240, 340) laminated to the thermoplastic layer towards the outer side of the textile electrode.

2. The textile electrode according to claim 1, wherein the thermoplastic layer comprises an outer thermoplastic layer (133, 233, 333) and an inner thermoplastic layer (132, 232, 332) having unmatching surfaces (138, 139; 238, 239) such that the closed, air-filled cavities occur between the outer and inner thermoplastic layers.

3. The textile electrode according to claim 2, wherein the inner thermoplastic layer (132, 232, 332) is thermoformed to rigidly provide the protruding portions.

4. The textile electrode according to claim 2 or 3, wherein the outer thermoplastic layer (133, 333) has a planar surface (138) to provide a planar textile layer for the electrode.

5. The textile electrode according to claim 2 or 3, wherein the outer thermoplastic layer (233) is thermoformed to provide an uneven textile layer (240) for the electrode.

6. The textile electrode according to any one of the preceding claims, wherein the textile electrode further comprises a protective layer (341, 461, 661) on an outer side of the textile layer (340, 460, 660).

7. The textile electrode according to any one of the preceding claims, wherein the textile electrode further comprises a conductive layer (160, 260, 360) on an inner side (181, 281, 381) of the thermoplastic layer.

8. The textile electrode according to any one of the preceding claims, further comprising a positioning layer (361) around the protruding portions on an inner side (181, 281, 381) of the thermoplastic layer (330) for holding the textile electrode in place during use.

9. A method for manufacturing a textile electrode (7) having an inner side (710) for contacting a skin surface and an outer side (720) for bordering with an environment, the method comprising:
- providing a first layer (791) having a textile sublayer (740) and an outer thermoplastic sublayer (733) laminated together;
- providing a second layer (792), comprising at least an inner thermoplastic sublayer (732);
- thermoforming (701) the second layer, such that an uneven surface is formed; and
- laminating (702) the first layer and the second layer together with the outer and inner thermoplastic sublayers facing each other, thereby obtaining closed, air-filled cavities (735) between the outer and inner thermoplastic sublayers.

10. The method according to claim 9, wherein the providing the first layer further comprises laminating (703) the outer thermoplastic sublayer and the textile sublayer together.

11. The method according to claim 9 or 10, further comprising providing a conductive layer (760) on the inner side of the electrode.

12. The method according to claim 11, wherein the second layer further comprises the conductive layer laminated (704) onto the inner thermoplastic sublayer.

13. The method according to claim 11, further comprising providing the conductive layer onto the thermoformed second layer.

14. The method according to any one of claims 9-13, further comprising laminating (705) a positioning layer around the protruding portions onto the inner side of the textile electrode for holding the textile electrode in place during use.

15. The method according to any one of claims 11-14 as long as dependent on claim 11, further comprising providing a snap fastener (371, 372, 445, 545, 645) through the layers of the textile electrode to provide an electrical contact from the outer side to the conductive layer.
